(19) Europäisches Patentamt
European Patent Office
Office européen des brevets

(11) Veröffentlichungsnummer: **0 409 147 B1**

(12) **EUROPÄISCHE PATENTSCHRIFT**

(45) Veröffentlichungstag der Patentschrift: **15.06.94**

(51) Int. Cl.[5]: **C07D 307/60**

(21) Anmeldenummer: **90113616.8**

(22) Anmeldetag: **16.07.90**

# (54) Verfahren zur Herstellung von Tetronsäurealkylestern.

(30) Priorität: **20.07.89 CH 2706/89**

(43) Veröffentlichungstag der Anmeldung:
**23.01.91 Patentblatt 91/04**

(45) Bekanntmachung des Hinweises auf die Patenterteilung:
**15.06.94 Patentblatt 94/24**

(84) Benannte Vertragsstaaten:
**CH DE FR GB LI**

(56) Entgegenhaltungen:
**EP-A- 0 247 320**
**DE-A- 2 025 570**
**DE-B- 2 845 037**

**Journal of the Chemical Society Perkin Transactions I 1987, LONDON Seiten 717 - 742; Andrew Pelter et al.: "Synthetic Routes to the Piperolides,Fadyenolides,Epoxypiperolides,and Related Compounds"**

**Chemical Reviews vol. 76, no. 5, 1976, Columbus Ohio Seiten 625 - 694; Y.S.Rao: "Recent Advances in the Chemistry of Unsaturated Lactones"**

(73) Patentinhaber: **LONZA AG**
**Gampel/Wallis**
**Geschäftsleitung Basel**
**CH-4002 Basel(CH)**

(72) Erfinder: **Duc, Laurent, Dr.**

**Chermignon (Kanton Wallis)(CH)**
Erfinder: **McGarrity, John, Dr.**
**Gemsweg 4**
**Visp (Kanton Wallis)(CH)**

(74) Vertreter: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von Tetronsäurealkylestern. Tetronsäurealkylester sind bekannte Verbindungen, die als wertvolle Zwischenprodukte, z.B. für Pharmaka, eingesetzt werden können. Vgl. dazu z.B. Pelter et al in J.Chem.Soc. Perkin Trans. I 1987 S.717 ff, oder Pelter et al in Tetrahedron Letters No.18 (1979) S.1627 ff.

Gegenstand der Erfindung ist ebenso die Verwendung der Tetronsäurealkylester zur Herstellung von Tetronsäure, einem weiteren wichtigen Baustein für zahlreiche Wirkstoffsynthesen.

Eine Herstellung von Tetronsäuremethylester ist z.B. bekannt aus der DE-PS 28 45 037, worin ein 4-Brom-3-methoxybut-2-encarbonsäureniederalkylester mit einer Lewis-Säure, z.B. Zinkdibromid, in einem organischen Lösungsmittel ringgeschlossen wird. Es werden Ausbeuten zwischen 70 und 80% beschrieben.

Aus ökologischer Sicht ist aber von Nachteil, dass bei diesem Verfahren Schwermetallkatalysatoren eingesetzt werden müssen, die nicht recycliert werden können. Das Nacharbeiten des Verfahrens mit dem entsprechenden 4-Chlor analog, hat zudem ergeben, dass die Ausbeute, wesentlich tiefer als angegeben, bei ca. 50% beziffert werden muss.

Es bestand daher die Aufgabe, ein Verfahren zu finden, das einerseits ökologisch unbedenklicher ist und andererseits hohe Ausbeuten eines qualitativ hochstehenden Produktes liefert.

Die Aufgabe konnte gelöst werden mit einem Verfahren nach Patentanspruch 1.

Ausgangsverbindungen des erfindungsgemässen Verfahrens sind die 4-Halogen-3-alkoxy-2-butencarbonsäureniederalkylester der allgemeinen Formel

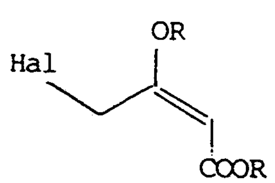

die auf einfache Weise, z.B. gemäss EP-Appl. 216 324 aus den grosstechnisch verfügbaren Halogenacetessigestern herstellbar sind.

In der allgemeinen Formel bedeutet Hal Chlor oder Brom und R eine Alkylgruppe mit 1 bis 6 C-Atomen.

Bevorzugt werden die 4-Chlor-3-($C_1$-$C_4$)-alkoxy-2-butencarbonsäure-($C_1$-$C_4$)-alkylester eingesetzt.

Der Ringschluss zum entsprechenden Tetronsäurealkylester der allgemeinen Formel

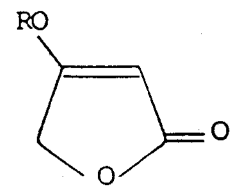

worin R die genannte Bedeutung hat, erfolgt bei einer Temperatur zwischen 190 und 260 °C, bevorzugt bei einer Temperatur zwischen 200 und 220 °C.

Das charakteristische Merkmal der Umsetzung ist, dass kein Lösungsmittel verwendet wird.

Mit Vorteil wird die Reaktion unter Ausschluss von Luftsauerstoff in einer Inertgasatmosphäre, vorzugsweise mit Stickstoff, durchgeführt.

Erfahrungsgemäss kann nach einer Reaktionsdauer von ca. 1 bis 6 h der entsprechende Tetronsäurealkylester aus dem Reaktionsgemisch, zweckmässig durch Destillation, isoliert werden.

Die erreichbaren Ausbeuten liegen in der Regel über 90%, die Reinheit der Produkte bei 99%.

Die erhaltenen Tetronsäurealkylester können erfindungsgemäss auf einfache Weise in praktisch quantitativer Ausbeute in die Tetronsäure überführt werden. Dazu wird der Tetronsäurealkylester einer sauren Hydrolyse, zweckmässig mit Chlorwasserstoff oder Bromwasserstoff in Essigsäure, unterworfen.

Als geeignete Hydrolysebedingungen haben sich bei der Anwendung von Chlorwasserstoff in Essigsäure ein Temperaturbereich von 50 bis 140 °C und ein Druck von 2 bis 8 bar herausgestellt. Bei der Anwendung von Bromwasserstoff kann bei Normaldruck und Temperaturen zwischen 30 und 120 °C gearbeitet werden.

Nach einer Reaktionszeit von ca. 3 bis 8 h kann die Tetronsäure auf übliche Weise in hervorragender Qualität isoliert werden.

Beispiel 1

Herstellung von Tetronsäuremethylester

195,1 g (1,19 mol) 4-Chlor-3-methoxy-2E-butencarbonsäuremethylester wurden in einer $N_2$-Atmosphäre während 5 h bei einer Temperatur von 200 bis 205 °C gerührt.

Das entstandene gasförmige Chlormethan wurde aufgefangen. Anschliessend wurde aus dem entstandenen Reaktionsgemisch bei 18 mbar und 130 bis 131 °C das Titelprodukt herausdestilliert. Man erhielt 127,4 g (92,5%) des Titelproduktes mit einer Reinheit von 98,2% (GC), Fp. 63 bis 64 °C.

Beispiel 2

Herstellung von Tetronsäureethylester

38,6 g (0,2 mol) 4-Chlor-3-ethoxy-2E-butencarbonsäureethylester wurden in $N_2$-Atmosphäre während $1\frac{1}{2}$ h bei einer Temperatur von 210 bis 220 °C gerührt.
Das entstandene Chlorethan wurde aufgefangen. Aus dem Reaktionsgemisch wurde anschliessend bei 16 mbar und 135 bis 137 °C das Titelprodukt herausdestilliert.
Man erhielt 22,5 g (87,4%) des Titelproduktes mit einem Gehalt von 99,5% (GC).

Beispiel 3

Herstellung von Tetronsäure-n-butylester

20,9 g (0,084 mol) 4-Chlor-3-n-butoxy-2E-butencarbonsäurebutylester wurden in $N_2$-Atmosphäre während $1\frac{1}{2}$ h bei einer Temperatur von 220 °C gerührt.
Das entstandene Chlorbutan wurde aufgefangen. Aus dem Reaktionsgemisch wurde bei 0,05 mbar und 87 bis 89 °C das Titelprodukt herausdestilliert.
Man erhielt 12,4 g (94%) des Titelproduktes mit einem Gehalt von 99% (GC).

Vergleichsbeispiel nach DE-PS 28 45 037

20 g (0,12 mol) 4-Chlor-3-methoxy-2E-butencarbonsäuremethylester wurden in 20 ml p-Xylol mit 0,1 g Zinkbromid vorgelegt und auf Rückflusstemperatur erhitzt. Der Reaktionsverlauf wurde mit GC kontrolliert.
Nach 7 h wurden nochmals 0,1 g Zinkbromid, nach 16 h nochmals 2 g Zinkbromid zugegeben.
Nach 24 h konnte ein 90%iger Umsatz an Edukt festgestellt werden (GC).
Darauf wurde das p-Xylol abdestilliert. Der Rückstand wurde extrahiert mit einem Gemisch von 12 ml Chloroform und 48 ml Ether.
Das Extrakt wurde darauf auf -27 °C gekühlt. Dabei fielen 7 g (51,1%) roher Tetronsäuremethylester aus. Dieser wurde bei 18 mbar und 130 bis 131 °C mittels Destillaton gereinigt.
Man erhielt dadurch 6,2 g (45,5%) Tetronsäuremethylester.

Beispiel 4

Herstellung von Tetronsäure

10 g (0,088 mol) Tetronsäuremethylester wurden in einem Autoklaven bei 5 °C in mit Chlorwasserstoff gesättigter Essigsäure vorgelegt. Bei einer Temperatur von 80 °C und einem Druck von 4 bar wurde das Reaktionsgemisch während 6 h gerührt.
Nach Abkühlen auf Raumtemperatur wurde zur Trockene eingedampft, der Rückstand in 30 ml Methylenchlorid aufgenommen, die Aufschlämmung auf 10 °C gekühlt, filtriert und das Produkt getrocknet.
Man erhielt 8 g (91%) des Titelproduktes mit einem Gehalt von 99,3% (HPLC), Fp. 135 bis 136 °C (Zersetzung).

**Patentansprüche**

1. Verfahren zur Herstellung von Tetronsäurealkylestern der Formel

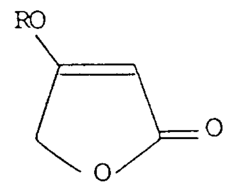

worin R eine Alkylgruppe mit 1 bis 6 C-Atomen bedeutet, dadurch gekennzeichnet, dass man einen 4-Halogen-3-alkoxy-2-butencarbonsäureniederalkylester der allgemeinen Formel

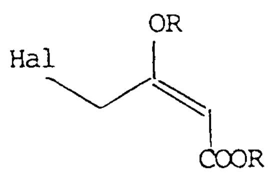

worin Hal Chlor oder Brom bedeutet und R die genannte Bedeutung hat, bei Temperaturen zwischen 190 und 260 °C ringschliesst.

2. Verfahren nach Patentanspruch 1, dadurch gekennzeichnet, dass bei Temperaturen zwischen 200 und 220 °C gearbeitet wird.

3. Verfahren nach mindestens einem der Patentansprüche 1 und 2, dadurch gekennzeichnet, dass die Reaktion unter Inertgasatmosphäre durchgeführt wird.

4. Verfahren nach mindestens einem der Patentansprüche 1 bis 3 dadurch gekennzeichnet, dass der resultierende Tetronsäurealkylester destillativ aus dem Reaktionsgemisch gewonnen wird.

5. Verwendung der Tetronsäurealkylester, hergestellt nach dem Verfahren nach mindestens

einem der Patentansprüche 1-4, zur Herstellung von Tetronsäure.

6. Verwendung nach Patentanspruch 5, dadurch gekennzeichnet, dass der Tetronsäurealkylester einer sauren Hydrolyse unterworfen wird.

7. Verwendung nach Patentanspruch 6, dadurch gekennzeichnet, dass die saure Hydrolyse mit Chlorwasserstoff oder Bromwasserstoff in Essigsäure durchgeführt wird.

## Claims

1. A process for the preparation of tetronic acid alkyl esters of the formula:

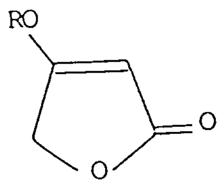

wherein R is an alkyl group having 1 to 6 carbon atoms, characterized in that a lower alkyl ester of 4-halogen-3-alkoxy-2-butenoic acid of the general formula:

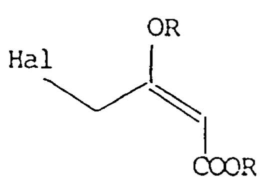

wherein Hal is chlorine or bromine and R has the above meaning, is cyclized at temperatures between 190 °C and 260 °C.

2. The process according to Claim 1 , characterized in that working takes place at temperatures between 200 °C and 220 °C.

3. The process according to at least one of Claims 1 and 2, characterized in that reaction is conducted under an inert gas atmosphere.

4. The process according to at least one of Claims 1 to 3, characterized in that the resulting tetronic acid alkyl ester is recovered from the reaction mixture by distillation.

5. Use of the tetronic acid alkyl esters prepared by the process according to at least one of Claims 1 to 4 in the preparation of tetronic acid.

6. Use according to Claim 5, characterized in that the tetronic acid alkyl ester is subjected to acid hydrolysis.

7. Use according to Claim 6, characterized in that acid hydrolysis is conducted with hydrogen chloride or hydrogen bromide in acetic acid.

## Revendications

1. Procédé pour la préparation d'alkylesters de l'acide tétronique de la formule

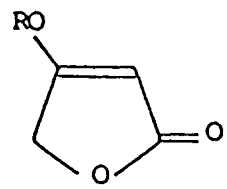

dans laquelle R signifie un groupe alkyle avec 1 à 6 atomes C, caractérisé en ce que l'on cyclise un alkylester inférieur de l'acide 4-halogène-3-alcoxy-2-butènecarboxylique de la formule générale

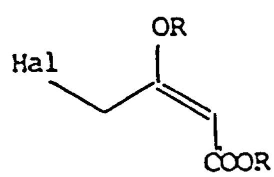

dans laquelle Hal signifie le chlore ou le brome et R a la signification indiquée, à des températures entre 190 et 260 °C.

2. Procédé selon la revendication 1, caractérisé en ce que l'on travaille à des températures entre 200 et 220 °C.

3. Procédé selon au moins l'une des revendications 1 et 2, caractérisé en ce que la réaction s'effectue sous atmosphère de gaz inerte.

4. Procédé selon au moins l'une des revendications 1 à 3, caractérisé en ce que l'alkylester de l'acide tétronique résultant est obtenu par distillation à partir du mélange réactionnel.

5. Utilisation de l'alkylester de l'acide tétronique, préparé selon le procédé selon au moins l'une

des revendications 1-4 pour la fabrication d'acide tétronique.

6. Utilisation selon la revendication 5, caractérisée en ce que l'alkylester de l'acide tétronique est soumis à une hydrolyse acide.

7. Utilisation selon la revendication 6, caractérisée en ce que l'hydrolyse acide est effectuée avec du chlorure d'hydrogène ou du bromure d'hydrogène dans l'acide acétique.